# EUROPEAN PATENT SPECIFICATION

(11) **EP 2 861 144 B1**
(45) Date of publication and mention of the grant of the patent: **16.08.2017**
(21) Application number: 13735106.0
(22) Date of filing: 18.06.2013
(51) Int. Cl.: A61B 5/15, A61B 5/151

(54) **LANCING DEVICE ENDCAP WITH INTERNAL DIAL-DRIVEN DEPTH ADJUST**
ENDKAPPE FÜR EINE LANZETTENVORRICHTUNG MIT INTERNER ANWÄHLBARER TIEFENEINSTELLUNG
BOUCHON D'EXTRÉMITÉ DE DISPOSITIF D'INCISION, POURVU D'UN AJUSTEMENT DE PROFONDEUR INTERNE COMMANDÉ PAR UN CADRAN

(30) Priority: 18.06.2012 US 201261660972 P
(43) Date of publication of application: 22.04.2015
(73) Proprietor: Facet Technologies, LLC, Kennesaw, GA 30144 (US)
(72) Inventor: HELLER, Kathleen, Glendale, CA 91207 (US)
(74) Representative: Maschio, Antonio
(86) International application number: PCT/US2013/046321
(87) International publication number: WO 2013/192179

(56) References cited:
- EP-A1- 1 625 824
- EP-A2- 1 074 219
- WO-A2-01/54750
- WO-A2-03/022130
- US-A1- 2004 127 818
- US-A1- 2006 271 084
- US-A1- 2007 083 222

## Description

### Cross-Reference to Related Application

This application claims the priority benefit of U.S. Provisional Patent Application Serial No. 61/660,972 filed June 18, 2012, the entirety of which is hereby incorporated herein by reference for all purposes.

### Technical Field

The present invention relates generally to the field of medical devices and procedures, and in particular to lancing devices for sampling blood or other bodily fluids. The invention more particularly relates to an endcap for use in conjunction with a lancing device that allows for adjustment to the penetration depth of a lancet.

### Background

Lancets and lancing devices are utilized for penetrating the skin of a human or animal subject a lancing site to obtain a sample of blood or other body fluid for medical testing, as in blood-typing or blood-glucose testing. Known lancing devices commonly include a housing containing a drive mechanism, a charging mechanism for energizing the spring or other drive means of the drive mechanism, and a release mechanism for releasing the drive mechanism upon actuation. A lancet is typically propelled by the drive mechanism from a retracted position within the housing to an extended position wherein a sharp tip portion of the lancet projects from the housing to prick the subject's skin at a desired lancing site.

Many lancing devices additionally include a depth-adjustment mechanism for adjusting the depth of penetration of the lancet projecting from the housing and into the subject's skin at a lancing site, in order to generate a desired sample size for testing. Previously known depth-adjustment mechanisms include the provision of an endcap that allows positional adjustment of an external component to change the length of the endcap and thereby vary the distance of spacing between a sampling site placed against the external face of the endcap and the extended position of the lancet. Other known depth adjustment mechanisms include repositionable stops within the lancing device housing that interact with a contact point of a lancet carrier to vary the stroke length of the lancet carrier.

Various disadvantages have been recognized in previously known mechanisms for providing adjustment to the depth of penetration of the lancet. For example, many known endcaps that control the depth of penetration by adjusting the position of the endcap in a helical motion relative to the housing provide no hard stop for restriction on the lancet travel, and may not provide as precise a control on penetration depth as a fixed stop does, particularly if the lancing stroke length of a device is inconsistent. Also, adjusting the endcap rotationally relative to the housing typically results in the endcap being roughly cylindrical-shaped, which can limit product design options. Operation of known adjustable endcap designs typically also changes the overall length of the lancing device and/or causes visible gaps between the cap and the housing body in certain states of adjustment, which may also be undesirable from a design standpoint.

Many known internal stop mechanisms for depth adjustment take up significant amounts of space within the housing of the lancing device. Excessive requirements for space within the housing may necessitate reducing the size of other components making them more fragile and prone to damage, or causing the lancing device to increase in size, resulting in a less convenient product design.
EP 1 625 824 discloses a lancing depth-adjustable lancing apparatus for moving a lancing element held in a housing in a lancing direction to lance skin with the lancing element. The lancing apparatus includes a lancing depth adjustment mechanism for adjusting lancing depth of the lancing element in the skin. The lancing depth adjustment mechanism includes a displacing member which is movable relative to the housing in the lancing or the retreating direction, a cover which is movable with the displacing member in the lancing or the retreating directions, and a stepped portion provided at the housing for restricting the movement of the lancing element in the lancing direction when the lancing element moves in the lancing direction.
WO 01/54750 discloses a lancet device having a body portion for firing a lancet and a tip portion. The tip portion includes a front assembly which includes a side wall portion, a skin-engaging portion at least partially defining a plane beyond which a lancet needle may extend, and a lancet stop element, a slot disposed in one of the sidewall portion and the lancet stop element, and at least one following element extending into the slot, wherein the lancet stop element is moveable towards and away from the plane in response to movement of the following element. A lancet device is also disclosed having a body portion for firing a lancet and a tip portion. The tip portion includes a front assembly which includes a side wall portion, a skin-engaging portion at least partially defining a plane beyond which a lancet needle may extend, and a lancet stop element, and at least one following element extending into the slot, wherein the lancet stop element is moveable towards and away from the plane in response to movement of the following element.
EP 1 074 219 discloses a lancer device that enables a user to draw blood from a patient and discard the used lancet without touching it. The device also has an adjustable tip for selecting the depth of stylet penetration into the patient and a triggering mechanism that utilizes a yoke latch and a leaf spring to discharge the lancet. The lancer also has a dampening feature to reduce vibrations when the lancet is moving.
US 2006/0271084 discloses a cap for a testing device. The cap includes a lancet movably mounted within the cap and a skin-engaging end having an opening that allows a portion of a needle of the lancet to pass therethrough. A test strip may be arranged on the cap.
WO 03/022130 discloses an arrangement of interfitting components for adjusting the penetration depth of a lancet and for stimulating an area surrounding an incision formed by penetration of a surface of the skin by the lancet, thereby facilitating the extraction of a sample of bodily fluid with a minimally invasive incision. The arrangement includes: a bottom end adapted to be applied to the surface of the skin, and a top end opposite the bottom end; a longitudinally moveable lancing member having a sharp end; a stop member setting a longitudinal travel distance of the lancing member; a bottom surface adapted to be applied to the surface of the skin, and an opening in the bottom surface through which the lancing member projects; a penetration depth defined by the distance between the end of the lancing device and the bottom surface; an adjusting mechanism for changing the penetration depth; and a stimulation mechanism for stimulating the area surrounding the incision and facilitating the extraction of the sample of bodily fluid.
US 2007/0083222 discloses a lancet device including a body and a holding member movably mounted within the body and comprising a front end and a rear end. The front end is configured to receive a lancet having the lancet needle. A lancet ejector and/or a biasing member are utilized. The biasing member is one of arranged within the holding member and biasing a mechanism for setting a trigger.

Accordingly, it can be seen that needs exist for improved lancing devices and depth adjustment mechanisms for a lancing device. It is to the provision of improved lancing devices and depth-adjust endcaps for lancing devices meeting these and other needs that the present invention is primarily directed.

### Summary

In accordance with the present invention, there is provided a depth adjuster as defined in claim 1.

In addition, further advantageous embodiments follow from the dependent claims.

The invention and advantageous embodiments of the invention will be understood with reference to the drawing figures and detailed description herein, and will be realized by means of the various elements and combinations as defined in the appended claims. It is to be understood that both the foregoing general description and the following brief description of the drawings and detailed description of the invention are exemplary and explanatory of preferred embodiments of the invention, and are not restrictive of the invention, as claimed.

### Brief Description of the Drawings

**FIGURE 1** shows a partial cross-sectional view of an adjustable depth endcap mounted to a lancing device according to an example embodiment of the present invention.
**FIGURE 2** shows a perspective view of the adjustable depth endcap of FIGURE 1.
**FIGURE 3** shows an assembly view of the adjustable depth endcap of FIGURE 1.
**FIGURE 4** shows a cross-sectional assembly view of the adjustable depth endcap of FIGURE 1.
**FIGURES 5** and **6** show side views of the adjustable depth endcap of FIGURE 1, showing the stop member positioned at both the minimum and maximum depth of penetration settings.

### Detailed Description of Example Embodiments

The present invention may be understood more readily by reference to the following detailed description of the invention taken in connection with the accompanying drawing figures, which form a part of this disclosure. It is to be understood that this invention is not limited to the specific devices, methods, conditions or parameters described and/or shown herein, and that the terminology used herein is for the purpose of describing particular embodiments by way of example only and is not intended to be limiting of the claimed invention.

Also, as used in the specification including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

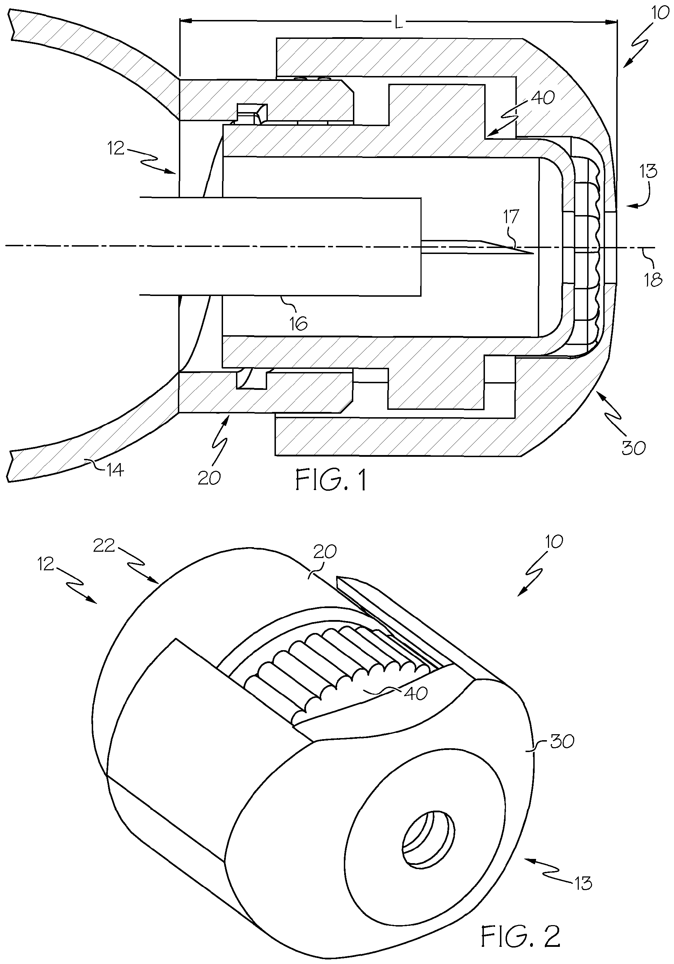

With reference now to the drawing figures, wherein like reference numbers represent corresponding parts throughout the several views, **Figures 1-6** show an endcap assembly or adjustable depth endcap 10 for a lancing device housing 14, the housing having a drive mechanism for a lancet 16 therein. The adjustable depth endcap 10 preferably is formed of substantially rigid bodies defining a longitudinal axis 18 extending from a first proximal end 12 to a second distal end 13, wherein the first proximal end is sized and/or shaped to attach to the lancing device housing 14. A substantially constant housing length L is preferably defined in the axial direction between the first proximal 12 and second distal 13 ends, regardless of the state of adjustment of the endcap.

The attachment of the adjustable depth endcap 10 can be accomplished by way of a releasable or permanent coupling such as for example a threaded screw coupling, a socket, snap fittings, friction fit, adhesive or welding, or one or more screws or other connectors, or the like; or the endcap 10 can comprise an integral portion of the overall housing of the lancing device 12.

The adjustable endcap 10 generally comprises a base member 20 defining the first proximal end 12, a cover 30 coupled to the base member and extending to the second distal end 13, and a dial or stop member 40 movably mounted therebetween. Rotation of the dial member 40 causes translational movement of the dial member along the longitudinal axis 18 in both a forward direction and a rearward direction depending on the direction of rotation (clockwise or counter-clockwise) of the dial 40.

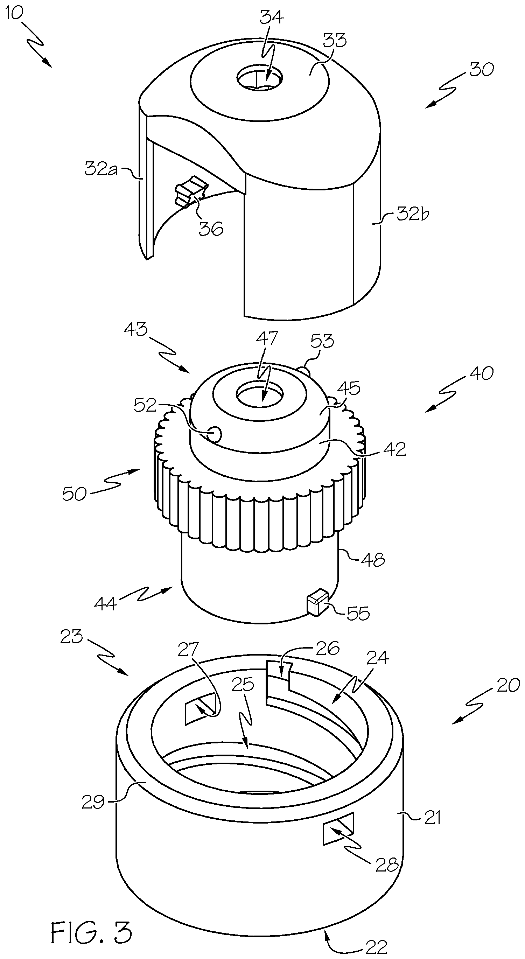

As shown in **Figures 3-4****,** the base member 20 defines a base body 21 extending from a first or mounting end 22 to a second or coupling end 23, wherein an opening 24 defining a substantially hollow interior portion is generally cylindrical-shaped to accommodate rotation of the dial member 40 therein. The outer surface of the base member body 21 can be shaped as desired; for example, a shape that allows for the base member to be better contoured to the shape of the housing of the lancing device 14, and need not be cylindrical as is typical with previously known rotationally adjusted endcaps. The opening 24 extending through the base member body 21 defines an interior surface having one or more helical threaded channels or guidance features 25 formed therein for movably mounting follower portions of the dial 40 thereto. Optionally, one or more axial entrance channels 26 may extend from the coupling end 23 into communication with the guide threads 25 to accommodate guide follower features of the dial 40 during assembly, as will be described below. The number and/or pitch of the helical guide channels 25 can vary to change the rotation-to-translation ratio of the dial 40 movably mounted thereto.

The base member body 21 also defines two external engagement openings or recesses 27, 28 adjacent the coupling end 23 for receiving engagement fingers 36,37 of each leg 32a, 32b of the cover member 30 therein, to provide a secure and substantially rigid connection of the cover 30 with the base member 20, while allowing for selective removal of the cover from the base member if desired. To accommodate coupling and decoupling, the engagement fingers 36, 37 of the cover 30 optionally comprise inclined faces, and/or the coupling end 23 of the base member can comprise a radiused or chamfered leading edge surface 29.

The cover 30 generally includes a body defining an exterior surface and an interior surface, with sidewall portions extending from a skin-contacting distal end face 33 to a proximal end generally opposite thereto. One or more cutout sections extending into the sidewalls of the cover body from the proximal end thereof divides the sidewall into one or more legs 32a, 32b. Flexure of the legs 32a, 32b allows the engagement features 36, 37 projecting therefrom to be snap fit onto the base 20 to couple with the engagement openings 27, 28 upon assembly. The interior of the cover 30 is substantially hollow and generally cylindrically-shaped to accommodate rotation of the dial member 40 therein. The exterior surface of the cover 30 can be shaped as desired; for example, a shape that allows for the cover 30 to be better contoured to the shape of the housing or the lancing device 14, and need not be cylindrical as is typical with previously known rotationally adjusted endcaps. The skin contacting end face 33 defines an orifice or opening 34 extending between the exterior surface and the interior surface through which a sharp tip portion 17 of the lancet projects to lance the skin of the subject at the sample site from which a sample of body fluid is expressed and collected. Preferably, the opening 34 is sized and shaped to permit only the sharp tip portion 17 to extend therethrough, thus preventing the body of the lancet 16 to extend therethrough.

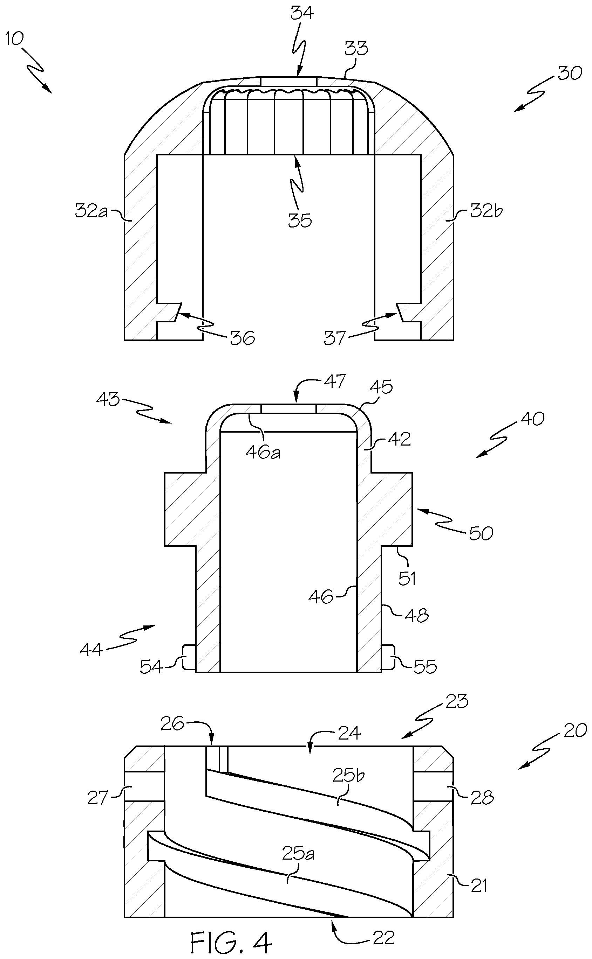

As depicted in **Figure 4**, the contact end 33 is convex, but alternatively can be planar or concave. The interior surface of the proximal end of the cover member 30 optionally comprises a plurality of positioning indents 35 surrounding the opening 34, which define a plurality of angular indexing positions of the dial 40 when mounted therein. The positioning indents 35 are oriented radially and lie in a generally ring-shaped pattern surrounding the opening 34, and optionally can be arranged in a regularly or an irregularly spaced pattern.

The dial member 40 comprises a generally cylindrically-shaped dial body 42 defining an interior surface and an exterior surface extending from a distal end 43 to a proximal end 44. An expanded diameter dial portion 50 having a ribbed or knurled outer circumferential surface extends outwardly from the dial body 42 between the proximal 44 and distal 43 ends. The dial portion 50 is externally accessible through sidewall openings between the legs 32 of the cover member 30, to provide a user interface for rotational adjustment of the dial member's position. Guide features or followers 54, 55 projecting from the outer surface of the dial body 42 adjacent the distal end 44 are configured to move within the helical guidance channels or threads 25 of the base member 20 upon rotation of the dial portion 50. The distal end 43 comprises a panel defining an orifice or opening 47 extending between an outer panel surface 45 and an inner panel surface 46. The inner panel surface 46 forms a stop surface against which the forward face of the lancet body 16 abuts to define the forward-most extent of its lancing stroke, allowing only the sharp tip portion 17 of the lancet to project through the opening 47. Optionally, one or more indexing beads or other positioning members 52, 53 project from or adjacent the outer panel surface 45 for cooperative interaction with the positioning indents 35 of the cover 30 to retain the dial 40 in a selected indexed position relative to the cover 30 and the housing of the lancing device 14, and thereby maintain a user-selected depth setting between uses.

The proximal end 44 of the dial member 40 preferably comprises an outer surface contour 48 adapted to be received within the opening 24 of the base member 20 with a free running or slight interference fit, allowing the dial 40 to rotate within the base member with light finger pressure applied by a user. The guide followers 54, 55 of the dial member 40 are cooperatively engaged with the helical guide channels 25 of the base member 20 to cause axial movement of the dial member 40 upon rotation of the dial portion 50. Axial movement of the dial member 40 may be limited by the extent of the guidance channels 25 within the base member 20, by stops on the guidance parts, and or by abutment of the dial member against the base member, the lancing device housing, and/or the cover.

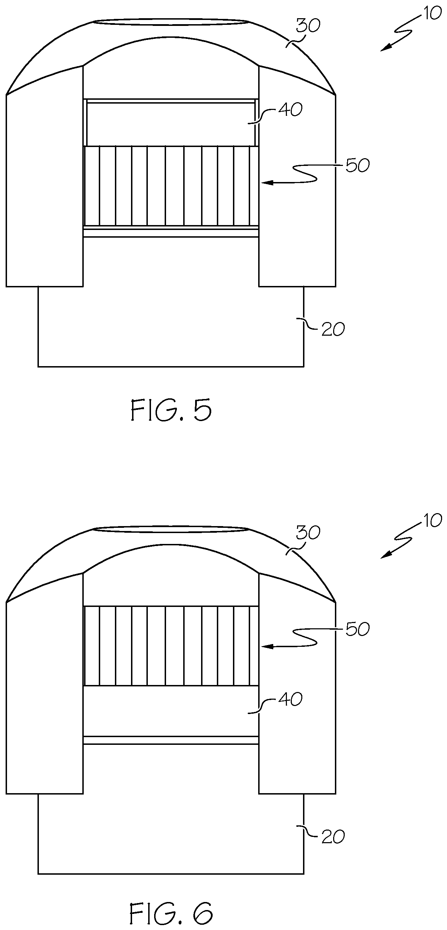

To assemble the adjustable depth endcap 10, the guide followers 54, 55 of the dial 40 are preferably inserted within the guidance channels 25 proximal the coupling end 23 of the base 20. For example, the guide follower 54 is placed in a receiver opening of a first guidance channel 25a and guide follower 55 is placed within the receiver opening 26 of the second guidance channel 25b. The dial 40 is then rotated in a first direction (e.g., clockwise) by actuating the dial portion 50 of the dial 40 until the bottom surface 51 contacts the coupling end 23 of the base member 20. With the legs 32a, 32b of the cover 30 extending towards the coupling end 23 of the base member 20, the legs 32a, 32b are pushed over the dial 40 and the cover 30 is further pressed to engage the engagement fingers 36, 37 within the engagement openings 27, 28 of the base member 20. Preferably, the legs 32a, 32b are configured to flex outwardly or inwardly to accommodate coupling thereto by fabrication of the cover from a material with sufficient elastic resilience. Once the cover 30 is securely mounted to the base member 20, the assembly of the adjustable depth endcap 10 is complete and ready for use; and the dial 40 is positioned such that the stop surface 46a is configured for providing a minimum depth of puncture (see **Figure 5**).

To provide additional or increased depth of puncture of the sharp tip portion 17 of the lancet 16 extending beyond the contact surface 33 of the cover 30, the dial portion 50 rotates in a second direction (e.g., counter-clockwise) and thereby causes the dial 40 to advance through one or more indexed angular positions as the stop surface 46a traverses along the longitudinal axis 18 (guided by traverse of the follower elements 54, 55 along the helical path of the guidance channels 25a, 25b) towards the second end 13, wherein each indexed rotational advancement provides incremental axial movement of the stop surface 46a to allow for a greater depth of puncture of the lancet 16 contacting the stop surface 46a. Continued rotation eventually causes the top end of the dial portion 50 to engage portions of the cover 30 near the legs, prohibiting further rotation (counter-clockwise) of the dial portion 50 and positioning the stop surface 46a to provide a maximum depth of puncture (see **Figure 6**). Thus, the stop surface 46a of the dial member is advanced toward or retracted away from the skin contacting face 33 of the cover to increase and decrease, respectively, the penetration depth of the lancet, by varying the position at which the stop surface 46a limits the forward extent of travel of the lancet relative to the skin contacting face of the cover. And because the cap 30 and base member 20 are fixed in position relative to the lancet housing, and the dial member 40 articulates within the cap and base, the overall length of the lancing device, including the endcap 10, remains substantially constant regardless of the penetration depth setting.

In alternative embodiments, one or more features referenced above can be arranged in different configurations to provide a substantially similar adjustable depth endcap. For example, the helical guidance features 25 of the base member 20 can form a portion of the cover 30, and the position indents 35 of the cover 30 can form a portion of the base member 20, thereby movably mounting the dial 40 to the cover and providing indexed rotation by interaction with portions of the base member 20. Similarly, raised helical threads can be provided on the base member 20, for engagement with a female follower recess on the dial member.

In further alternative embodiments, the dial grip can be formed such that the dial portion 50 extends beyond the periphery of the cover 30 or around the same wherein the legs 32a, 32b can extend within the dial 40 and couple to the base member 20 while allowing rotation and subsequent translational movement of the dial 40 therebetween. Optionally, the legs 32a, 32b can form a portion of the base member 20 and extend through or around the dial and couple to the cover 30.

In further alternative embodiments, the stop surface 46a on the dial can be configured to contact and stop a lancet holder carrying the lancet 16 instead of the lancet itself as described above. For example, the base member 20 can form a portion of the housing of the lancet carrier 14 and the dial 40 can permanently engage the base member 20 for movement within the guide channels 25, wherein the dial 40 is preferably sized and/or shaped to provide selective engagement with a portion of the lancet holder of the lancing device as it is propelled along the lancing stroke.

While the invention has been described with reference to preferred and example embodiments, it will be understood by those skilled in the art that a variety of modifications, additions and deletions are within the scope of the invention, as defined by the following claims.

## Claims

1. A depth adjuster (10) for adjusting the penetration depth of a lancet (16) out of a housing (14), the depth adjuster (10) comprising:
a longitudinal axis (18) extending between a proximal end (12) of the depth adjuster (10) and a distal end (13) of the depth adjuster (10);
a hollow base (20) for securing to or forming a portion of to the housing (14), the base (20) comprising an interior surface and at least one guidance feature (25) extending along the hollow base interior surface; and
a hollow stop member (40) comprising at least one follower element (54, 55) slidably supported within the at least one guidance feature (25) in the hollow base interior surface, the stop member (40) configured to translationally travel along the longitudinal axis (18) with respect to the hollow base (20);
a cap (30) to receive the base (20) and the stop member (40), the cap (30) comprising an aperture configured to axially align with the longitudinal axis; **characterized in that** the base (20) comprises at least one engagement receiver (27, 28), and the cap (30) comprises at least one engagement finger (36, 37) for removable engagement with the base engagement receiver (27, 28); and that the cap (30) comprises an access opening configured to provide access to the stop member (40).

2. The depth adjuster of Claim 1, wherein the at least one guidance feature (25) comprises at least one helical guide (25).

3. The depth adjuster of Claim 2, further comprising at least one entrance channel (26) providing access to the at least one helical guide (25).

4. The depth adjuster of Claim 2, wherein rotation of the stop member (40) along the at least one helical guide (25) causes the stop member (40) to translate along the longitudinal axis (18).

5. The depth adjuster of Claim 1, wherein the stop member (40) comprises a dial (50) comprising a grip surface.

6. The depth adjuster of Claim 5, wherein the dial grip surface comprises an expanded diameter.

7. The depth adjuster of Claim 1, wherein the stop member (40) comprises a connector end (44) and a stop end (43), the at least one follower element (54, 55) being positioned along the connector end (44).

8. The depth adjuster of Claim 7, wherein the stop end (43) comprises a stop surface (46a) comprising an orifice (47) extending therethrough, the stop surface (46a) being configured to temporarily engage the lancet (16).

9. The depth adjuster of Claim 1, wherein the stop member (40) is configured to translate along the longitudinal axis (18) with respect to the cap (30).

## Patentansprüche

1. Tiefeneinsteller (10) zum Einstellen der Eindringtiefe einer Lanzette (16) aus einem Gehäuse (14) heraus, wobei der Tiefeneinsteller (10) umfasst:
eine Längsachse (18), die sich zwischen einem proximalen Ende (12) des Tiefeneinstellers (10) und einem distalen Ende (13) des Tiefeneinstellers (10) erstreckt;
eine Hohlbasis (20) zum Befestigen von oder Ausbilden eines Abschnitts davon an dem Gehäuse (14), wobei die Basis (20) eine innere Oberfläche und mindestens ein Führungsmerkmal (25) umfasst, das sich entlang der Hohlbasis der inneren Oberfläche erstreckt; und
ein Hohlstoppbauteil (40), umfassend mindestens ein Folgeelement (54, 55), gleitfähig getragen in dem mindestens einen Führungsmerkmal (25) in der inneren Oberfläche der Hohlbasis, wobei das Stoppbauteil (40) zur translatorischen Bewegung entlang der Längsachse (18) hinsichtlich der Hohlbasis (20) ausgelegt ist;
eine Kappe (30) zum Aufnehmen der Basis (20) und des Stoppbauteils (40), wobei die Kappe (30) eine Öffnung, ausgelegt zum axialen Ausrichten mit der Längsachse, umfasst;
**dadurch gekennzeichnet, dass** die Basis (20) mindestens einen Eingriffaufnehmer (27, 28) umfasst, und die Kappe (30) mindestens einen Eingrifffinger (36, 37) zum entfernbaren Eingriff mit dem Eingriffaufnehmer (27, 28) der Basis umfasst; und
dass die Kappe (30) eine Zugangsöffnung, ausgelegt zum Bereitstellen des Zugangs zu dem Stoppbauteil (40), umfasst.

2. Tiefeneinsteller nach Anspruch 1, wobei das mindestens eine Führungsmerkmal (25) mindestens eine Spiralführung (25) umfasst.

3. Tiefeneinsteller nach Anspruch 2, weiterhin umfassend mindestens einen Eintrittskanal (26) zum Bereitstellen des Zugangs zu der mindestens einen Spiralführung (25).

4. Tiefeneinsteller nach Anspruch 2, wobei Rotation des Stoppbauteils (40) entlang der mindestens einen Spiralführung (25) das Stoppbauteil (40) veranlasst, sich entlang der Längsachse (18) zu verschieben.

5. Tiefeneinsteller nach Anspruch 1, wobei das Stoppbauteil (40) eine Einstellscheibe (50), umfassend eine Griffoberfläche, umfasst.

6. Tiefeneinsteller nach Anspruch 5, wobei die Einstellscheiben-Griffoberfläche einen breiteren Durchmesser umfasst.

7. Tiefeneinsteller nach Anspruch 1, wobei das Stoppbauteil (40) ein Verbinderende (44) und ein Stoppende (43) umfasst, wobei das mindestens eine Folgeelement (54, 55) entlang des Verbinderendes (44) positioniert ist.

8. Tiefeneinsteller nach Anspruch 7, wobei das Stoppende (43) eine Stoppoberfläche (46a), umfassend eine Öffnung (47), die sich dort hindurch erstreckt, umfasst, wobei die Stoppoberfläche (46a) zum zeitweiligen Eingriff mit der Lanzette (16) ausgelegt ist.

9. Tiefeneinsteller nach Anspruch 1, wobei das Stoppbauteil (40) zum Verschieben entlang der Längsachse (18) hinsichtlich der Kappe (30) ausgelegt ist.

## Revendications

1. Dispositif de réglage de profondeur (10) pour régler la profondeur de pénétration d'un bistouri (16) hors d'un logement (14), le dispositif de réglage de profondeur (10) comprenant :
un axe longitudinal (18) s'étendant entre une extrémité proximale (12) du dispositif de réglage de profondeur (10) et une extrémité distale (13) du dispositif de réglage de profondeur (10) ;
une base creuse (20) pour fixation à ou formation d'une portion du logement (14), la base (20) comprenant une surface intérieure et au moins une particularité de guidage (25) s'étendant le long de la surface intérieure de base creuse ; et
un élément formant butée creuse (40) comprenant au moins un élément suiveur (54, 55) supporté de manière coulissante à l'intérieur de l'au moins une particularité de guidage (25) dans la surface intérieure de base creuse, l'élément formant butée (40) étant configuré pour se déplacer en translation le long de l'axe longitudinal (18) par rapport à la base creuse (20) ;
un capuchon (30) pour recevoir la base (20) et l'élément formant butée (40), le capuchon (30) comprenant une ouverture configurée pour s'aligner de façon axiale sur l'axe longitudinal ;
**caractérisé en ce que**
la base (20) comprend au moins un récepteur de mise en prise (27, 28), et le capuchon (30) comprend au moins un doigt de mise en prise (36, 37) pour une mise en prise amovible avec le récepteur de mise en prise de base (27, 28) ; et
**en ce que** le capuchon (30) comprend une ouverture d'accès configurée pour fournir un accès à l'élément formant butée (40).

2. Dispositif de réglage de profondeur selon la revendication 1, dans lequel l'au moins une particularité de guidage (25) comprend au moins un guide hélicoïdal (25).

3. Dispositif de réglage de profondeur selon la revendication 2, comprenant en outre au moins un canal d'entrée (26) fournissant un accès à l'au moins un guide hélicoïdal (25).

4. Dispositif de réglage de profondeur selon la revendication 2, dans laquelle la rotation de l'élément formant butée (40) le long de l'au moins un guide hélicoïdal (25) amène l'élément formant butée (40) à subir une translation le long de l'axe longitudinal (18).

5. Dispositif de réglage de profondeur selon la revendication 1, dans lequel l'élément formant butée (40) comprend un sélecteur rotatif (50) comprenant une surface de saisie.

6. Dispositif de réglage de profondeur selon la revendication 5, dans lequel la surface de saisie de sélecteur rotatif comprend un diamètre étendu.

7. Dispositif de réglage de profondeur selon la revendication 1, dans lequel l'élément formant butée (40) comprend une extrémité de connecteur (44) et une extrémité de butée (43), l'au moins un élément suiveur (54, 55) étant positionné le long de l'extrémité de connecteur (44).

8. Dispositif de réglage de profondeur selon la revendication 7, dans lequel l'extrémité de butée (43) comprend une surface de butée (46a) comprenant un orifice (47) s'étendant à travers cette dernière, la surface de butée (46a) étant configurée pour temporairement mettre en prise le bistouri (16).

9. Dispositif de réglage de profondeur selon la revendication 1, dans lequel l'élément formant butée (40) est configuré pour subir une translation le long de l'axe longitudinal (18) par rapport au capuchon (30).
